(19)

**Europäisches Patentamt**

**European Patent Office**

**Office européen des brevets**

(11) **EP 4 158 655 B1**

(12) **EUROPEAN PATENT SPECIFICATION**

(45) Date of publication and mention
of the grant of the patent:
**17.12.2025 Bulletin 2025/51**

(21) Application number: **21727893.6**

(22) Date of filing: **26.05.2021**

(51) International Patent Classification (IPC):
**G16H 50/20** *(2018.01)* **G16H 50/30** *(2018.01)*
**G16H 50/70** *(2018.01)*

(52) Cooperative Patent Classification (CPC):
**G16H 50/30; G16H 50/20; G16H 50/70**

(86) International application number:
**PCT/EP2021/064062**

(87) International publication number:
**WO 2021/239816 (02.12.2021 Gazette 2021/48)**

(54) **METHOD OF DETERMINING A RISK OF MORTALITY OF A CANCER PATIENT, METHOD OF ASSESSING AN ANTI-CANCER THERAPY, METHOD OF SELECTING CANCER PATIENTS FOR TREATMENT**

VERFAHREN ZUR BESTIMMUNG DES STERBLICHKEITSRISIKOS EINES KREBSPATIENTEN, VERFAHREN ZUR BEURTEILUNG EINER ANTIKREBSTHERAPIE, VERFAHREN ZUR AUSWAHL VON KREBSPATIENTEN ZUR BEHANDLUNG

PROCÉDÉ DE DÉTERMINATION DU RISQUE DE MORTALITÉ D'UN PATIENT ATTEINT DE CANCER, PROCÉDÉ D'ÉVALUATION D'UNE THÉRAPIE ANTICANCÉREUSE, PROCÉDÉ DE SÉLECTION DE PATIENTS ATTEINTS DE CANCER EN VUE D'UN TRAITEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2020 EP 20177361**

(43) Date of publication of application:
**05.04.2023 Bulletin 2023/14**

(73) Proprietor: **F. Hoffmann-La Roche AG**
**4070 Basel (CH)**

(72) Inventors:
• **RUETTINGER, Dominik**
**82377 Penzberg (DE)**
• **BAUER-MEHREN, Anna**
**82377 Penzberg (DE)**
• **BECKER, Tim**
**47877 Willich (DE)**

(74) Representative: **J A Kemp LLP**
**80 Turnmill Street**
**London EC1M 5QU (GB)**

(56) References cited:
• **ELIZABETH KVALE ET AL: "History of cancer and mortality in community-dwelling older adults", CANCER EPIDEMIOLOGY, ELSEVIER, AMSTERDAM, NL, vol. 35, no. 1, 13 July 2010 (2010-07-13), pages 30 - 36, XP028141949, ISSN: 1877-7821, [retrieved on 20100720], DOI: 10.1016/ J.CANEP.2010.07.011**
• **SYDÓ NÓRA ET AL: "Effect of Cardiorespiratory Fitness on Co-Morbidities and Mortality in Never, Past, and Current Smokers", AMERICAN JOURNAL OF CARDIOLOGY, CAHNERS PUBLISHING CO., NEWTON, MA, US, vol. 122, no. 10, 21 August 2018 (2018-08-21), pages 1765 - 1772, XP085535235, ISSN: 0002-9149, DOI: 10.1016/J.AMJCARD.2018.08.012**

**Description**

The present invention relates to methods of determining a risk of mortality of a cancer patient. The methods find application in contexts where quantification of such risk is beneficial, such as in assessment of anti-cancer therapy and/or in selection of patients for patient management regimes, treatment plans, or clinical trials.

Factors influencing life expectancy are important in public health. In oncology, prediction of patient survival is instrumental for optimal patient management. By understanding which variables are prognostic, we gain insights into disease biology, and are able to improve design, conduct, and data analysis of clinical trials and real-world data. Current research on prognostic factors in oncology is largely based on comparatively small sample sizes and studies one risk factor at a time. Examples of such research include the following:

- Banks, E. et al. Erectile dysfunction severity as a risk marker for cardiovascular disease hospitalisation and all-cause mortality: a prospective cohort study. PLoS Med. 10, e1001372 (2013).
- Hu, F. B. et al. Adiposity as compared with physical activity in predicting mortality among women. N. Engl. J. Med. 351, 2694-2703 (2004).
- McGee, D. L., Liao, Y., Cao, G. & Cooper, R. S. Self-reported health status and mortality in a multiethnic US cohort. Am. J. Epidemiol. 149, 41-46 (1999).
- Thun, M. J. et al. Alcohol consumption and mortality among middle-aged and elderly U.S. adults. N. Engl. J. Med. 337, 1705-1714 (1997).
- Tota-Maharaj, R. et al. Coronary artery calcium for the prediction of mortality in young adults <45 years old and elderly adults >75 years old. Eur. Heart J. 33, 2955-2962 (2012).

Existing prognostic scores in common use are constructed from a relatively small number of risk factors. Such scores include the following:

- the Royal Marsden Hospital Score (RMHS) (Nieder, C. & Dalhaug, A. A new prognostic score derived from phase I study participants with advanced solid tumours is also valid in patients with brain metastasis. Anticancer Res. 977-9 (2010)).
- the international prognostic index (IPI) (International Non-Hodgkin's Lymphoma Prognostic Factors Project. A predictive model for aggressive non-Hodgkin's lymphoma. N. Engl. J. Med. 329, 987-994 (1993)).
- the IMDC risk model (Ko, J. J. et al. The International Metastatic Renal Cell Carcinoma Database Consortium model as a prognostic tool in patients with metastatic renal cell carcinoma previously treated with first-line targeted therapy: a population-based study. Lancet Oncol. 16, 293-300 (2015)).
- the Glasgow prognostic score (Kinoshita, A. et al. The Glasgow Prognostic Score, an inflammation based prognostic score, predicts survival in patients with hepatocellular carcinoma. BMC Cancer 52 (2013) doi:10.1186/1471-2407-13-52).

The recent UK biobank initiative (Sudlow, C. et al. UK Biobank: An Open Access Resource for Identifying the Causes of a Wide Range of Complex Diseases of Middle and Old Age. PLoS Med. 12, e1001779 (2015)) constitutes an important addition to data availability. The initiative was used by Ganna and Ingelsson (Ganna, A. & Ingelsson, E. 5-year mortality predictors in 498 103 UK Biobank participants: a prospective population-based study. The Lancet 386, 533-540 (2015)) to investigate life expectancy in a population-based sample of ~500,000 participants and construct a mortality risk score outperforming the Charlson comorbidity index (Charlson, M. E., Pompei, P., Ales, K. L. & C.Ronald, M. A new method of classifying prognostic comorbidity in longitudinal studies: Development and validation. J Chronic Dis. 373-383 (1987) doi:10.1016/0021-9681(87)90171-8).

ELIZABETH KVALE ET AL: "History of cancer and mortality in community-dwelling older adults", CANCER EPIDEMIOLOGY, ELSEVIER, AMSTERDAM, NL, vol. 35, no. 1, 13 July 2010, pages 30-36 discloses a study in which propensity scores for cancer were used to assemble a cohort of participants with and without cancer who were balanced on baseline characteristics. Cox regression models were used to determine the association between cancer and all-cause mortality among matched patients, and to identify independent predictors of mortality among unmatched cancer patients.

It is an object of the invention to improve quantitative determination of mortality risk.

According to an aspect of the invention, there is provided the computer-implemented method of determining a risk of mortality of a cancer patient according to claim 1.

Thus, a method is provided in which a risk of mortality is determined and output. The output is provided in the form of a numerical score. The numerical score may be output as data or shown on a display. The determination is performed using a model based on a specific selection of parameters that provides an optimized balance between high sensitivity and specificity on the one hand and manageable computational and data collecting demands on the other. As data included herein demonstrates, the core choice of 16 model parameters was found to provide sensitivity and specificity significantly above what has been observed to be possible in alternative prior art prognostic scores. Improved performance is feasible by including still further model parameters but the improvement that is possible is relatively small in comparison with the advantage already obtained relative to the alternative prior art prognostic scores. The selection of the core 16 model parameters is thus demonstrated to provide the good balance between sensitivity and specificity on the one hand and manageable computational and data collecting demands on the other.

The model was developed by the inventors performing prognostic modelling of overall survival (OS) on cohort data from the Flatiron Health database (122,694 patients). The inventors validated their results in two independent clinical studies. They examined demographic, clinical, hematology and blood chemistry parameters (focusing on routinely collected data), cancer diagnosis, and alongside real-world mortality as endpoint and assessed survival time from the first line of treatment.

The model parameters represent selected factors reflecting both tumor biology and patient characteristics and the determined score is shown to have a prognostic value that strongly outperforms contemporary risk scores. The score can be used to improve risk stratification, patient matching, and interpretation of clinical study results for early- and late-stage oncology drug development.

The inventors found that the determined score correlated not only with OS but also with particular early study dropout. Hence, using the determined score to exclude very high-risk patients may help protect patients from unnecessary exposure to study procedural burden and potential adverse events.

The inventors also found a surprisingly clear increase in the determined score towards death. During dose escalation in first-in-human studies it is a plausible hypothesis that the determined score will increase under inefficient drug dosages or treatments, but will stay stable or even improve under effective treatment. In this way, group level analysis of the time course of the determined score by treatment arm or drug dosage can give valuable insights on potential drug efficacy. Increase of determined score might, in combination with death events, be used as a surrogate end-point in clinical studies.In an embodiment, the mathematical model models the relationship determined from training data between the risk of mortality and values of the model parameters (i)-(xvi) and at least the following parameters:

(xvii) smoking history;
(xviii) number of metastatic sites;
(xix) platelet level in blood;
(xx) calcium level in serum or plasma;
(xxi) glucose level in blood;
(xxii) ratio of lymphocytes to leukocytes in blood;
(xxiii) level of bilirubin in serum or plasma;
(xxiv) level of monocytes in blood;
(xxv) level of oxygen saturation in arterial blood; and
(xxvi) body mass index.

In an embodiment, the mathematical model models the relationship determined from training data between the risk of mortality and values of the model parameters (i)-(xxvi) and at least the following parameter: (xxvii) alanine aminotransferase enzymatic activity level in serum or plasma.

This particular selection of 27 model parameter has been found by the inventors to provide a particularly beneficial balance of performance. The inventors observe significantly improved separation of Kaplan-Meier survival curves in comparison with RMHS.

In an embodiment, the mathematical model models the relationship determined from training data between the risk of mortality and values of the model parameters (i)-(xxvii) and at least the following parameters:

(xxviii) level of eosinophils in blood; and
(xxix) diastolic blood pressure.

The inventors have found that adding these two extra model parameters provides improvement in sensitivity and/or specificity without excessive increases in computational and/or data collecting burden.

The inventors have demonstrated that the approach is robust to missingness in data at levels up to about 10 missing values of parameters. Missing values may be replaced, for example, by mean values of the parameters of interest, taken from the training data for the model. In an embodiment, the patient data comprises values for each of at least 16 of the model parameters where at least model parameters (i)-(xxvi) are used. Where only model parameters (i)-(xvi) are used, the approach will typically be robust to missingness in data at levels up to about 3 or 4 missing values of parameters.

In an embodiment, the model comprises a weighted sum of deviations in the patient data from mean values of the model parameters in the training data, and the model is formed by determining the weightings from the training data. Forming the model in this way is computationally efficient and provides high performance. The trained model can be represented in a compact form and applied with modest computational resources.

Embodiments of the invention will now be described, by way of example only, with reference to the accompanying drawings.

Figure 1 is a flow chart depicting a framework for methods of determining a risk of mortality according to embodiments of the disclosure.

Figure 2 is a plot visualizing performance of a model of a risk of mortality with respect to increasing regularization strength.

Figure 3 depicts the importance of model parameters in terms of HR estimates and corresponding confidence intervals; protective parameters have an HR less than 1, indicating that higher levels of the parameter are beneficial; detrimental parameters have an HR greater than 1, and imply higher risk with higher variable value (abbreviations are used - see below).

Figure 4 depicts Kaplan-Meyer curves of overall survival (OS) for high/low RMHS score.

Figure 5 depicts Kaplan-Meyer curves of overall survival (OS) for a highest risk 10% model score group vs remaining 90% model score group for scores determined using methods of the present disclosure.

Figure 6 depicts Kaplan-Meyer curves of overall survival (OS) for score deciles from low (decile 1) to high (decile 10) risk for scores determined using methods of the present disclosure.

Figure 7 depicts a ROC curve for 3-month survival for a phase I study and RMHS, with AUC = 72.3.

Figure 8 depicts a ROC curve for 3-month survival for a phase I study and the present model, with AUC = 84.1.

Figure 9 depicts a ROC curve for 3-month survival for a phase III study and RMHS, with AUC = 64.2.

Figure 10 depicts a ROC curve for 3-month survival for a phase III study and the present model, with AUC = 81.7.

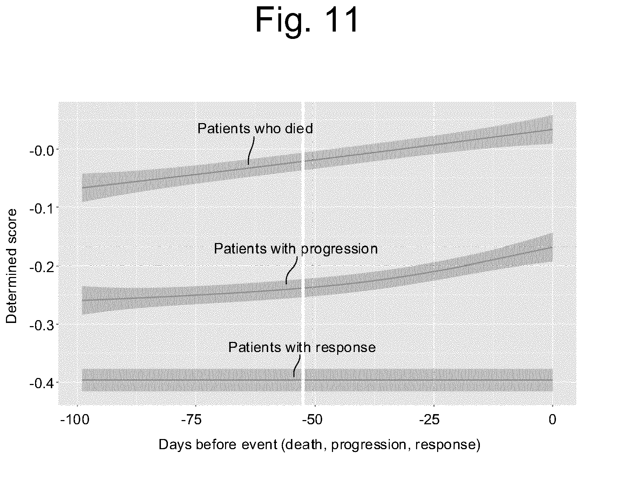

Figure 11 depicts results from longitudinal monitoring of model score by a response group in OAK phase III clinical study and landmark analysis. The x-axis corresponds to different time points. The date of occurrence of an outcome event is the right-most point. To the left, the time course prior to the event is depicted (100 days). Only data from patients for which at least 100 days were observable contribute to the figure. The y-axis corresponds to the determined score (group averages). Each curve represents one of three outcome groups (death, progression, response as join of complete and partial response).

Figure 12 is a Kaplan-Meyer for landmark analysis under landmark at 145 days (=75% of patients).

Figure 13 is a graph corresponding to the graph of Figure 3 except using a reduced model containing a selected 16 model parameters (referred to as ROPRO 16).

Figure 14 is bar chart comparing performance between models of the present disclosure having different numbers of model parameters and prior art alternative prognostic scores. Two comparisons are shown: Cindex (comparing general performance for OS prediction) and AUC (comparing performance to predict 12 week survival).

Figure 15 is a bar chart comparing performance between a model of the present disclosure (with 27 model parameters, referred to as ROPRO) and prior art alternative prognostic scores using a database covering 20 clinical studies from phase 1 to phase 3 across different cancer indications.

Figure 16 is a bar chart comparing performance between two different models of the present disclosure (with 27 and 26 model parameters) and prior art alternative prognostic scores using a database covering 20 clinical studies from phase 1 to phase 3 across different cancer indications.

Figure 17 is a graph depicting a Kaplan-Meyer Survival Analysis of OAK study. The study showed that Atezolizumab treatment results in a statistically significant and clinically relevant improvement in OS vs Docetaxel in 2L/3L NSCLC. This survival analysis confirms the finding.

Figure 18 is a graph depicting a delta ROPRO analysis of the two arms of the study referred to in relation to Figure 17 above. The ROPRO score is significantly increasing in patients in the Docetaxel group compared to Atezolizumab group indicating that the delta ROPRO is indicative of treatment efficacy. This trend is in addition seen earlier than in the survival analysis.

The following abbreviations are used herein:

**Abbreviations**

| ALP | Alkaline phosphatase |
| ALT | Alanine aminotransferase |
| AST | Aspartate aminotransferase |
| BMI | Body mass index |
| CI | Confidence interval |

| CLL | Chronic lymphocytic leukemia |
|---|---|
| CRC | Colorectal cancer |
| CSF1 | Colony stimulating factor-1 |
| DLBCL | Diffuse large B-cell carcinoma |
| ECOG | Eastern Cooperative Oncology Group (performance status) |
| EHR | Electronic health record |
| HCC | Hepatocellular carcinoma |
| HER2 | Human epidermal growth factor receptor 2 |
| HR | Hazard ratio |
| IPI | International prognostic index |
| KM | Kaplan-Meier |
| LDH | Lactate dehydrogenase |
| NLR | Neutrophil-to-lymphocyte ratio |
| NSCLC | Non-small-cell lung cancer |
| OS | Overall survival |
| PDL1 | Programmed death-ligand 1 |
| PFS | Progression-free survival |
| RMHS | Royal Marsden Hospital score |
| RCC | Renal cell carcinoma |
| RoPro | Roche Prognostic Score |
| rSq | Generalized r-squared ($r^2$) |
| SCLC | Small-cell lung cancer |
| TNM | TNM system (tumor, node, metastasis) |

In the following, methods are described for determining a risk of mortality of a cancer patient. The determined risk may be represented as a score (i.e. a quantitative measure, such as a real number). This score may be referred to herein as a Roche Prognostic Score (RoPro). The model that is used to obtain the score may be referred to herein as a RoPro model.

Methods of the present disclosure may be computer-implemented. Each step of the disclosed methods may be performed by a computer in the most general sense of the term, meaning any device capable of performing the data processing steps of the method, including dedicated digital circuits. The computer may comprise various combinations of computer hardware, including for example CPUs, RAM, SSDs, motherboards, network connections, firmware, software, and/or other elements known in the art that allow the computer hardware to perform the required computing operations. The required computing operations may be defined by one or more computer programs. The one or more computer programs may be provided in the form of media or data carriers, optionally non-transitory media, storing computer readable instructions. When the computer readable instructions are read by the computer, the computer performs the required method steps. The computer may consist of a self-contained unit, such as a general-purpose desktop computer, laptop, tablet, mobile telephone, smart device (e.g. smart TV), etc. Alternatively, the computer may consist of a distributed computing system having plural different computers connected to each other via a network such as the internet or an intranet.

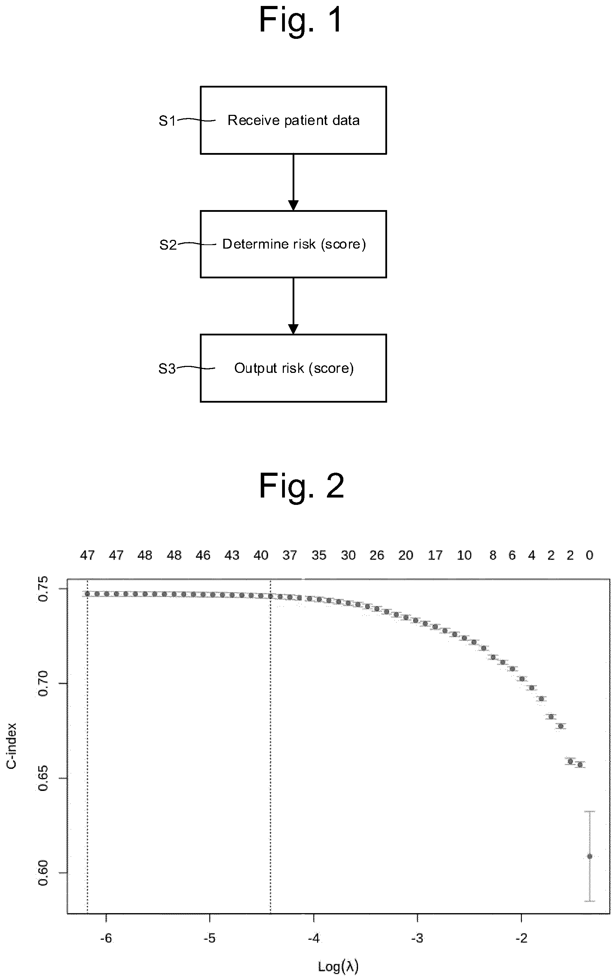

Figure 1 is a flow chart depicting a framework for a method of determining a risk of mortality of a cancer patient according to the present disclosure. In step S1, the method comprises receiving patient data representing information about a cancer patient. The patient data may be received by a computer configured perform the method using any of the various known ways of providing data to a computer. In step S2, a mathematical model of mortality risk is used to determine a risk of mortality of the cancer patient based on the received patient data. In step S3, the determined risk of mortality is output, for example as data and/or directly onto a local display.

In some embodiments, the mathematical model models a relationship determined from training data between the risk of mortality and values of a selected group of model parameters. The model is thus a trained model. The training data may comprise historical patient data from a database. Any of the various known ways of training a mathematical model using training data may be used.

The inventors used training data obtained from the Flatiron Health database (flatiron: https://flatiron.com). They performed a retrospective cohort analysis using electronic health records (EHRs) from the Flatiron Health database. The Flatiron Health database contains demographically and geographically diverse longitudinal data from over 280 oncology clinics in the USA. Institutional review board approval of the study protocol was obtained before study conduct and included a waiver of informed consent. Data from the February 2020 data release were extracted, including patient demographics and clinical data (e.g., type of cancer, disease stage, comorbidities, medication prescriptions, routine blood biomarkers). Patient records with missing first line of treatment information and patient records/variables with high missing data rates were excluded. Missing values in the final analysis data set were imputed.

In some embodiments, the model is formed by performing multivariable Cox regression analysis on the training data for a plurality of subjects, preferably at least 1000 subjects.

The inventors used a Cox proportional hazard model (Cox DR. Regression Models and Life-Tables. Journal of the Royal Statistical Society. Series B (Methodological) Vol. 34). Risk of mortality was investigated as overall survival (OS). Survival time was calculated from the start of the first line of treatment (defined as $T_0$) to the event of "death" (death from all causes), as coded in the real-world mortality table (Stock, C., Mons, U. & Brenner, H. Projection of cancer incidence rates and case numbers until 2030: A probabilistic approach applied to German cancer registry data (1999-2013). Cancer Epidemiol 57, 110-119 (2018)) in the Flatiron Health database. For cohorts comprising only patients with advanced/metastatic disease, the first line of systemic treatment for advanced/metastatic disease is classed as first line in the database. Censored follow-up times were computed as days elapsed from $T_0$ to the date of the patient's last documented clinic contact. The patient's last available pre-$T_0$ measurement was used for modelling. A time limit of 30 days prior to $T_0$ was applied. See Connolly, J. G., Schneeweiss, S., Glynn, R. J. & Gagne, J. J. Quantifying bias reduction with fixed-duration versus all-available covariate assessment periods. Pharmacoepidemiol. Drug Saf. 28, 665-670 (2019).

The inventors discovered from early experimentation with a smaller data version from 2018 that there was very little evidence that the routine variables available in Flatiron Health are of varying importance across cohorts. The inventors therefore built a pan-indication score (representing a determined risk of mortality).

For model comparison, the following were considered for censored data and are referred to below:

- the generalized-$r^2$ (Kalbfleisch, JD & Prentice, RL. The statistical analysis of failure time data, Second Edition. (Wiley Series in Probability and Statistics, 2002)).
- the concordance index (C-index) (Steck, H., Krishnapuram, B., Dehing-Oberije, C., Lambin, P. & Raykar, V. C. On Ranking in Survival Analysis: Bounds on the Concordance Index. in Advances in Neural Information Processing Systems 20 (eds. Platt, J. C., Koller, D., Singer, Y. & Roweis, S. T.) 1209-1216 (Curran Associates, Inc., 2008)).
- the ROC area under the curve (ROC-AUC) (Blanche, P, Dartigues, JF & Jacquim-Gadda, H. Estimating and comparing time-dependent areas under receiver operating characteristic curves for censored event times with competing risks).

All analyses were conducted with the statistical analysis package R (R Core Team (2013). R: A language and environment for statistical computing. R Foundation for Statistical Computing, Vienna, Austria. URL http://www.R-project. org/).

Model selection was performed via a Family-wise error rate controlled (FWER-controlled) backward selection procedure on the Cox regression model, starting from 46 initial variables. The variable with least impact on model performance was iteratively removed until all remaining parameters yielded a measurable improvement in concordance index and generalized-$r^2$ and were significant at $\alpha 1 = 0.05/46 = 0.0011$ (Bonferroni-correction). By construction, the procedure controls the family-wise error rate at $\alpha = 0.05$, i.e., all parameters are significant after adjustment for multiple testing. In parallel, the inventors derived a regularized Lasso model with 10-fold cross-validation (Simon N, Friedman J, Hastie T, Tibshirani R. Regularization Paths for Cox's Proportional Hazards Model via Coordinate Descent. J. Stat. Softw. Artic. 2011;39(5):1-13). The model performance is visualized in Figure 2 with respect to increasing regularization strength (larger $\lambda$). On top of the plot, the number of selected variables is depicted (indication variables and non-indication variables). $\lambda$ was selected so as to extract the most regularized model with a C-index within one standard error (right vertical dashed line in Figure 2) of the best performing model (left vertical dashed line).

For continuous variables, the Cox model yields hazard ratios (HRs) per unit of the investigated variable (model parameter); e.g., the HR for age is per age difference of one year. For better comparability across variables, the "4SD-HR", the HR of a patient with a particularly high variable value (equal to mean + 2 standard deviations (SD)) versus one with a low value (mean - 2SD), is provided as descriptive measure.

Overall, 122,694 patients across 17 cancer-type-specific cohorts were eligible for analysis. Table 1 summarizes the patient characteristics.

**Table 1: Final RoPro Cox Regression Model (Overall Survival) and Description of RoPro Variables**

| Table 1. Final RoPro Cox Regression Model (Overall Survival) and Description of RoPro Variables | | | | | | | |
|---|---|---|---|---|---|---|---|
| Variable | transformation[1] | P | HR[2] | 4SD-HR[3] | 4SD-HR (lasso)[4] | unit | mean[5] |
| age | 0 | 4,50E-112 | 1.01 [1.009; 1.01] | 1,519 | 1,474 | years | 67,15 |

(continued)

| Table 1. Final RoPro Cox Regression Model (Overall Survival) and Description of RoPro Variables | | | | | | | |
|---|---|---|---|---|---|---|---|
| | | | | | | | |
| Variable | transfor mation[1] | P | HR[2] | 4SD-HR[3] | 4SD-HR (lasso)[4] | unit | mean[5] |
| sex | 0 | 3,18E-60 | 1.152 [1.133; 1.172] | 1,327 | 1,250 | none[7] | 0,50 |
| smoking | 0 | 6,06E-39 | 1.216 [1.18; 1.252] | 1,311 | 1,433 | none[8] | 0,58 |
| Number of metastatic sites | 0 | 2,84E-28 | 1.029 [1.024; 1.034] | 1,165 | 1,091 | none | 0,90 |
| Hgb | 0 | 2,69E-66 | 0.959 [0.955; 0.964] | 0,725 | 0,824 | g/dL | 12,09 |
| urea nitrogen | 1 | 3,02E-83 | 1.21 [1.187; 1.234] | 1,398 | 1,312 | mg/dL | 2,78 |
| platelets | 0 | 3,06E-42 | 0.999 [0.999; 0.999] | 0,776 | 0,971 | 10*9/L | 267,42 |
| calcium | 1 | 2,46E-54 | 2.892 [2.529; 3.307] | 1,313 | 1,078 | mg/dL | 2,23 |
| glucose | 1 | 1,23E-04 | 1.063 [1.03; 1.096] | 1,062 | 1,002 | mg/dL | 4,73 |
| lymphocytes-leukocytes-ratio | 1 | 4,75E-08 | 0.89 [0.853; 0.928] | 0,746 | 0,548 | % | 2,95 |
| ALP | 1 | 4,33E-99 | 1.209 [1.188; 1.231] | 1,475 | 1,409 | U/L | 4,58 |
| protein | 0 | 1,13E-43 | 0.991 [0.99; 0.992] | 0,779 | 0,861 | g/L | 68,89 |
| ALT | 1 | 3,07E-08 | 0.95 [0.933; 0.968] | 0,873 | –[9] | U/L | 3,01 |
| albumin | 0 | <2.2*E-308 | 0.961 [0.959; 0.963] | 0,440 | 0,471 | g/L | 37,85 |
| bilirubin | 1 | 1,32E-26 | 1.085 [1.069; 1.102] | 1,196 | 1,154 | mg/dL | -0,77 |
| chloride | 0 | 9,76E-120 | 0.976 [0.974; 0.978] | 0,673 | 0,699 | mmol/ L | 101,43 |

(continued)

| Table 1. Final RoPro Cox Regression Model (Overall Survival) and Description of RoPro Variables | | | | | | | |
|---|---|---|---|---|---|---|---|
| | | | | | | | |
| Variable | transfor mation[1] | P | HR[2] | 4SD-HR[3] | 4SD-HR (lasso)[4] | unit | mean[5] |
| monocytes | 1 | 2,14E-40 | 1.113 [1.095; 1.13] | 1,287 | 1,100 | 10*9/L | -0,55 |
| eosinophils-leukocytes-ratio | 1 | 8,36E-32 | 0.947 [0.939; 0.956] | 0,816 | 0,908 | % | 0,31 |
| LDH | 2 | 4,43E-60 | 3.412 [2.945; 3.953] | 1,391 | 1,247 | U/L | 1,69 |
| heart rate | 1 | 7,20E-84 | 1.528 [1.464; 1.594] | 1,378 | 1,265 | bpm | 4,40 |
| SBP | 1 | 4,48E-47 | 0.671 [0.636; 0.709] | 0,796 | 0,834 | mmHg | 4,85 |
| oxygen | 0 | 1,98E-21 | 0.979 [0.975; 0.984] | 0,856 | 0,864 | % | 96,49 |
| ECOG performance status | 0 | 8,14E-214 | 1.222 [1.207; 1.238] | 1,714 | 1,762 | none | 0,84 |
| NLR[10] | 1 | 3,00E-08 | 1.095 [1.06; 1.13] | 1,457 | 1,068 | % | 1,16 |
| BMI | 0 | 1,96E-27 | 0.843 [0.817; 0.869] | 0,843 | 0,888 | kg/m$^2$ | 3,30 |
| AST-ALT-ratio | 1 | 3,99E-29 | 1.14 [1.114; 1.167] | 1,262 | 1,229 | % | 0,09 |
| TumorStage | 0 | 3,33E-72 | 1.084 [1.075; 1.094] | 1,417 | 1,432 | none[11] | 3,10 |
| eosinophils[12] | 0 | - | - | - | 0,993 | 10*9/L | 0,16 |
| DBP[12] | 0 | - | - | - | 0,997 | mmHg | 74,18 |

(continued)

| Table 1. Final RoPro Cox Regression Model (Overall Survival) and Description of RoPro Variables | | | | | | | |
|---|---|---|---|---|---|---|---|
| | | | | | | | |
| Variable | transfor mation[1] | *P* | HR[2] | 4SD-HR[3] | 4SD-HR (lasso)[4] | unit | mean[5] |
| | | | | | | | |

[1]Indicates if log-transformation was performed, "2" indicates $\log^2$-transformation. All measures are given after the transformation.

[2]Hazard ratio on scale of variable (= per measurement unit, if applicable after log-transformation), together with 95% confidence interval

[3]Hazard ratio of patient with parameter value of mean+2SD compared to patient with value of mean-2SD [4]4SD-HR of lasso model

[5]Mean value in Flatiron Health data (after transformation, if applicable)

[6]Standard deviation in Flatiron Health data (after transformation, if applicable)

[7]Coded 1=male, 0=female

[8]Coded 1=History of smoking, 0= No history or unknown

[9]Not included in lasso model

[10]Neutrophils-lymphocytes-ratio

[11]TNM coding

[12]Included only in lasso model

After quality control, data were available for 46 general measurements, overall missing rate prior to imputation 21%, and 99 further cohort-specific measures (cytogenetics, biomarkers). Median survival time was 19.0 months (95% confidence interval [CI] 18.8-19.2).

*ROPRO*

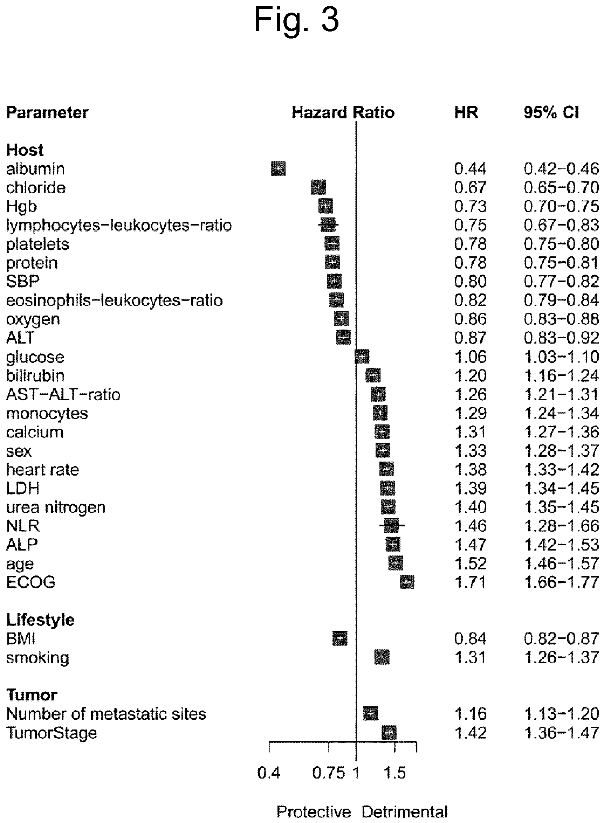

Initial unadjusted analysis identified 44 variables significantly associated with OS. The backward selection model selected 27, while the cross-validated Lasso model selected 28 independently contributing variables, of which 26 coincided (Table 1). In both cases, plots of the concordance index by the number of included variables showed no evidence for an earlier saturation of the models with fewer variables (Figure 2). Prognostic risk scores implied by the two modelling approaches had a correlation of $r^2$=0.993, demonstrating the interchangeability and stability of the modeling. All further result descriptions making reference to the "ROPRO" or "RoPro" model or score refer to the model with 27 independently contributing in multivariable modelling (Table 1, Figure 3). The model has a global shrinkage factor of 0.998, the difference between apparent $r^2$ and Nagelkerkes / Cragg & Uhlers pseudo-$r^2$ according was as small as $1.0 \times 10^{-6}$ and a small absolute margin of error (0.0017) for 3-months survival. Thus, all modelling conditions recently required in the following are met: Riley, R. D. et al. Minimum sample size for developing a multivariable prediction model: PART II - binary and time-to-event outcomes. Stat. Med. 38, 1276-1296 (2019).

The 27 variables found to be predictive may be used as the model parameters for the model of mortality risk. In the specific example described here, the resulting determined risk is output as a prognostic score referred to as a RoPro score. The score is based on a weighted sum of the patients' differences from the respective reference means over the variables (see Table 1 for details). An example of the RoPro score may be determined from the following: 0.00948(age-67.15) + 0.14162(sex-0.502) + 0.19521(smoking-0.576) + 0.02833(Number of metastatic sites-0.897) - 0.04178(Hgb-12.087) + 0.19097(urea nitrogen-2.777) - 6e-04(platelets-267.423) + 1.0619(calcium-2.231) + 0.06098(glucose-4.733) - 0.11658(lymphocytes-leukocytes-ratio-2.953) + 0.19019(ALP-4.582) - 0.00896(protein-68.888) - 0.05113(ALT-3.008) - 0.03988(albumin-37.851) + 0.08189(bilirubin-(-0.773)) - 0.02462(chloride-101.434) + 0.10671(monocytes-(-0.548)) - 0.0543(eosinophils-leukocytes-ratio-0.307) + 1.22733(LDH-1.694) + 0.42372(heart rate-4.402) - 0.39878(SBP-(4.846)) - 0.02083(oxygen-96.487) + 0.20066(ECOG-0.84) + 0.0905(NLR-1.156) - 0.17076(BMI-3.301) + 0.13122(AST-ALT-ratio-0.092) + 0.081(TumorStage-3.098).

The 27 model parameters are as follows:

(i) age;
(ii) gender;
(iii) smoking history;
(iv) number of metastatic sites;

(v) haemoglobin or haematocrit level in blood;

(vi) urea nitrogen level in serum or plasma;

(vii) platelet level in blood;

(viii) calcium level in serum or plasma;

(ix) glucose level in blood;

(x) ratio of lymphocytes to leukocytes in blood;

(xi) alkaline phosphatase enzymatic activity level in serum or plasma;

(xii) protein level in serum or plasma;

(xiii) level of albumin in serum or plasma;

(xiv) level of bilirubin in serum or plasma;

(xv) chloride or sodium level in serum or plasma;

(xvi) level of monocytes in blood;

(xvii) ratio of eosinophils to leukocytes in blood;

(xviii) lactate dehydrogenase enzymatic activity level in serum or plasma;

(xix) heart rate;

(xx) systolic blood pressure;

(xxi) level of oxygen saturation in arterial blood;

(xxii) Eastern cooperative oncology group (ECOG) performance status;

(xxiii) ratio of neutrophils to lymphocytes in blood;

(xxiv) body mass index;

(xxv) ratio of aspartate aminotransferase enzymatic activity level in serum or plasma to alanine aminotransferase enzymatic activity level in serum or plasma;

(xxvi) TNM classification of tumor stage; and

(xxvii) alanine aminotransferase enzymatic activity level in serum or plasma.

In the example above, the model of mortality risk models a relationship determined from training data between the risk of mortality and values of all of the model parameters (i)-(xxvii), which is found to provide particularly high performance. However, acceptable performance may also be obtaining using a smaller number of the available model parameters.

In some embodiments, the model models the relationship determined from the training data between the risk of mortality and the values of at least 16, optionally at least 18, optionally at least 20, optionally at least 22, optionally at least 24, optionally all of, the model parameters (i)-(xxvi). In some embodiments, the model models the relationship determined from the training data between the risk of mortality and the values of at least 16, optionally at least 18, optionally at least 20, optionally at least 22, optionally at least 24, of the model parameters (i)-(xxvii).

In some embodiment, the following additional model parameters are used: (xxviii) level of eosinophils in blood; and (xxix) diastolic blood pressure.

The use of these extra model parameters increases the model complexity and data requirements, but may provide further performance improvements. In embodiments of this this type, the model may model the relationship determined from the training data between the risk of mortality and the values of at least 16, optionally at least 18, optionally at least 20, optionally at least 22, optionally at least 24, optionally all of, the model parameters (i)-(xxix).

For a particular patient, it may be that values for some of the model parameters used by the model are not available. The inventors have found that the model can still provide a reliable score in these circumstances up to a missingness of about 10 values. Thus, the patient data should preferably comprise values for each of at least 16, optionally at least 18, optionally at least 20, optionally at least 22, optionally at least 24, of the model parameters used by the model. Alternatively, the patient data comprises values for all of the model parameters (i)-(xxvi). Alternatively, the patient data comprises values for all of the model parameters (i)-(xxvii). Alternatively, the patient data comprises values for all of the model parameters (i)-(xxix).

*ROPRO16*

As mentioned above, in some embodiments the mathematical model models the relationship between the risk of mortality and values of at least 16 model parameters. In one class of embodiment, the 16 model parameters include the following:

(i) age;

(ii) gender;

(iii) haemoglobin or haematocrit level in blood;

(iv) urea nitrogen level in serum or plasma;

(v) alkaline phosphatase enzymatic activity level in serum or plasma;

(vi) protein level in serum or plasma;

(vii) level of albumin in serum or plasma;

(viii) chloride or sodium level in serum or plasma;

(ix) ratio of eosinophils to leukocytes in blood;

(x) lactate dehydrogenase enzymatic activity level in serum or plasma;

(xi) heart rate;

(xii) systolic blood pressure;

(xiii) Eastern cooperative oncology group (ECOG) performance status;

(xiv) ratio of neutrophils to lymphocytes in blood;

(xv) ratio of aspartate aminotransferase enzymatic activity level in serum or plasma to alanine aminotransferase enzymatic activity level in serum or plasma; and

(xvi) TNM classification of tumor stage.

In the case where only these 16 model parameters are used, the model may be referred to as the 16 variable reduced model or "ROPRO16" model.

In some embodiments, the model is expanded to 26 parameters by including the following parameters:

(xvii) smoking history;

(xviii) number of metastatic sites;

(xix) platelet level in blood;

(xx) calcium level in serum or plasma;

(xxi) glucose level in blood;

(xxii) ratio of lymphocytes to leukocytes in blood;

(xxiii) level of bilirubin in serum or plasma;

(xxiv) level of monocytes in blood;

(xxv) level of oxygen saturation in arterial blood; and

(xxvi) body mass index.

In some embodiments, the model is expanded to 27 parameters by including the following parameter: (xxvii) alanine aminotransferase enzymatic activity level in serum or plasma.

In some embodiments, the model is expanded to 29 parameters by including the following parameters:

(xxviii) level of eosinophils in blood; and

(xxix) diastolic blood pressure.

*Example uses of the determined mortality risk*

As mentioned above, in some embodiments the determination of the mortality risk comprises calculating a numerical score representing the mortality risk. In some embodiments, the determination of the mortality risk further comprises comparing the calculated score to one or more predetermined threshold values, or to calculated scores for other cancer patients. For example, it may be determined that the mortality risk is "high" if the calculated score is above a predetermined threshold value, or above an average score calculated for a selected group of cancer patients. The mortality risk may be determined as "low" if the calculated score is below a predetermined threshold value, or below an average score calculated for a selected group of cancer patients.

The score thus provides a practical quantitative tool that represents a physical state of a patient. The score is obtained the same way for different patients or for the same patient at different times. The score can thus be used as an objective, quantitative basis for comparing different patients between each other and/or for comparing states of the same patient at different times. The score thus provides a quantitative tool which can be used in various ways to support objective decision making in relation to providing health care for patients and/or to facilitate extraction of objective information from studies that involve the patients.

In some embodiments, the model comprises a weighted sum of deviations in the patient data from mean values of the model parameters in the training data, and the model is formed by determining the weightings from the training data.

In an embodiment, the model is formed by: assigning a respective weighting, $w_i$, to each of the model parameters, and determining a respective mean, $m_i$, of values of each model parameter over the training data. The determination of the mortality risk may then comprise calculating a numerical score according to the following formula:

$$\text{score} = \sum_i w_i(m_{ij} - m_i)$$

where $w_i$ is the weighting of the $i$-th model parameter, $m_i$ is the mean of the $i$-th model parameter, and $m_{ij}$ is the value of the $i$-th model parameter for a $j$-th cancer patient for whom the score is to be calculated.

In an embodiment, each of the weightings $w_i$ comprises a natural logarithm of the Hazard Ratio (HR), such that the score is given as follows:

$$\text{score} = \sum_i \ln(HR(x_i)(m_{ij} - m_i)$$

where $HR(x_i)$ is the HR estimated for variable $i$, $m_i$ is the variable mean in the training data (e.g. Flatiron Health database), and $m_{ij}$ is the value of patient $j$ at variable $i$, $i \in I$.

The determined risk of mortality (e.g. as a score) may be used in various situations as mentioned above.

In one class of embodiment, a method of assessing an anti-cancer therapy that uses the determined score may be provided. The method comprises determining a risk of mortality of a patient at plural different times while the patient is receiving the anti-cancer therapy, by performing the method of determining a mortality risk according any of the embodiments of the present disclosure at each of the plural times, and analysing the resulting determined risks to determine an efficacy of the anti-cancer therapy.

Alternatively or additionally, a method of selecting cancer patients for treatment with an anti-cancer therapy that uses the determined score may be provided. The method comprises determining a risk of mortality of a candidate patient using the method of determining a mortality risk according to any of the embodiments of the present disclosure. The determining risk is then used to decide whether to select each candidate patient.

Further examples of applications of the determined risk (e.g. score) are discussed below.

*Performance Evaluation of RoPro*

The performance of an example implementation of the method of determining a risk of mortality is discussed in this section. The example implementation uses the 27 model parameters (i)-(xxvii). In this case, the determined risk is represented as a score that is referred to as a RoPro score or simply "RoPro".

Individual patient RoPro scores (derived by inputting their measurements for each of the variables into the formula) ranged from -3.49 to 4.12, 99% in [-2.21; 2.09]. Higher scores indicate higher risk.

The model in this example was found to strongly outperform the RMHS with respect to all model performance indicators ($r^2$=0.32, C-index=0.747, 3-month-AUC=0.82 vs. $r^2$=0.03, C-index=0.54, 3-month-AUC=0.58).

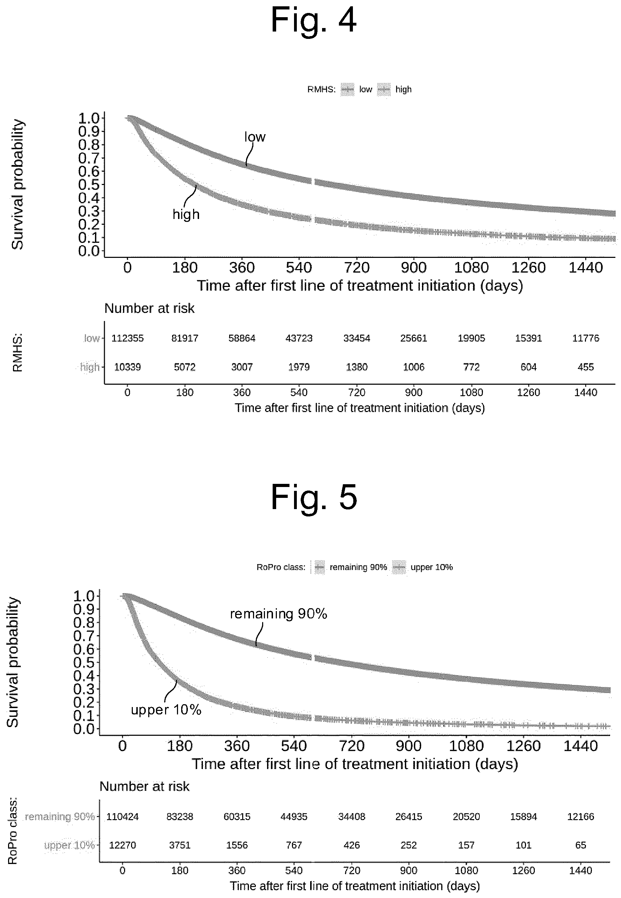

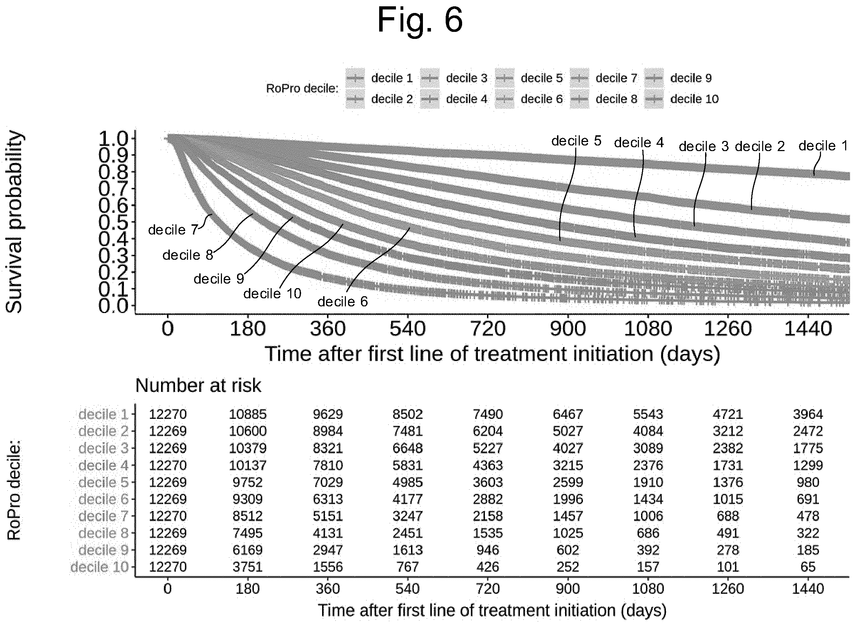

Figures 4-6 present a comparison of RMHS and RoPro. Figure 4 shows a clear separation of survival curves according to high/low RMHS (HR 2.31; 95% CI 2.26-2.36). Figure 5 depicts patients with the highest 10% RoPro scores versus the remaining 90%, and shows improved separation of the survival curves (HR 4.85; 95% CI 4.75-4.94). Moreover, subdividing the sample into ten subgroups of equal size but increasing RoPro (deciles) showed clear separation of the respective Kaplan-Meier survival curves (Figure 6). Median survival was clearly separated along the deciles, with a median survival of 2,975 days in the lowest versus 114 days in the highest decile. The highest-risk patients (RoPro score>1.18) had an HR of 27.6 (95% CI 26.37-28.88), $P$<2.23×$10^{-308}$, compared with the lowest-risk group (score< -1.29).

The RoPro score showed strong performance in all 17 cohorts, based on highly consistent variable (model parameter) estimate sizes and directions across cohorts. With cohort-specific re-estimated variable weights, ln($HR(x_i)$, strong improvement of model performance was seen for chronic lymphocytic leukemia (CLL) ($r^2$=0.13, C-index=0.704, 3-month-AUC=0.82 for general RoPro in CLL; $r^2$=0.17, C-index=0.74, 3-month-AUC=0.84 for CLL-specific RoPro).

*Model validation of RoPro in clinical trial populations*

For validation purposes, the (RoPro) model was applied retrospectively, e.g. without re-estimation of the variable weights, to patients from two independent clinical studies. Here, the RoPro score, as a single variable, was fitted using Cox regression. The first analysis was based on a phase I first-in-human study, BP29428 (NCT02323191), which investigated the combination of emactuzumab, a monoclonal antibody targeting the receptor for colony stimulating factor-1 (CSF1) (Gomez-Roca, C. A. et al. Phase I study of emactuzumab single agent or in combination with paclitaxel in patients with advanced/metastatic solid tumors reveals depletion of immunosuppressive M2-like macrophages. Ann. Oncol. 30, 1381-1392 (2019)) and atezolizumab, a monoclonal antibody targeting PDL1, in patients (n=216) with locally advanced or metastatic solid tumors not amenable to standard treatment. The second analysis used data from the phase III OAK study, which evaluated efficacy and safety of atezolizumab monotherapy versus single agent docetaxel in participants (n=1187) with locally advanced or metastatic non-small-cell lung cancer (NSCLC) after failure of platinum-containing chemotherapy (Rittmeyer, A. et al. Atezolizumab versus docetaxel in patients with previously treated non-small-cell lung cancer (OAK): a phase 3, open-label, multicentre randomised controlled trial. Lancet 389, 255-265 (2017)).

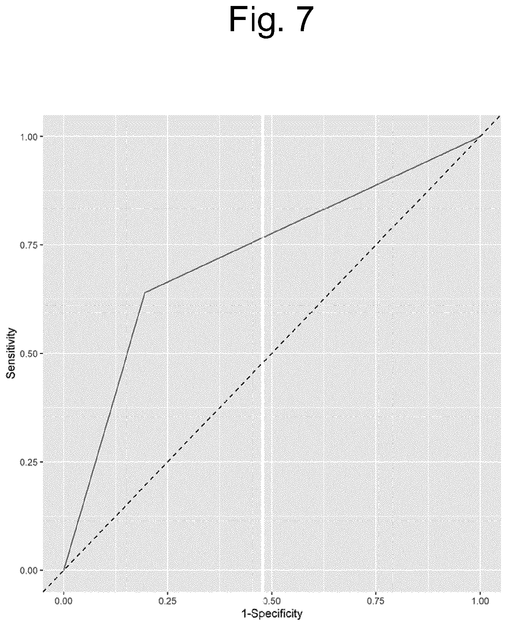

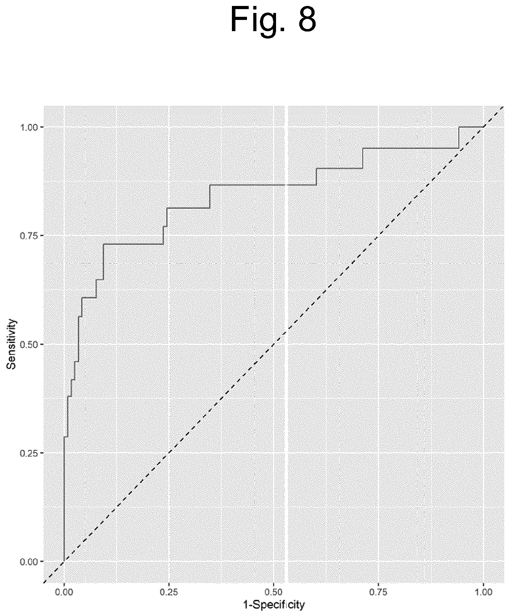

First, the inventors could replicate the correlation of RoPro with OS in the phase I study BP29428 (n=216, $P$=6.08x10$^{-15}$, r2=0.23, C-index=0.81). Patients with RoPro>1.18 (cut-off equal to 90%-quantile in Flatiron Health, n=8) had particular poor OS prognosis (HR 11.29; 95% CI 5.52-23.12). Three-month-survival ROC-AUC values were comparable to that in Flatiron Health (Figures 7 and 8).

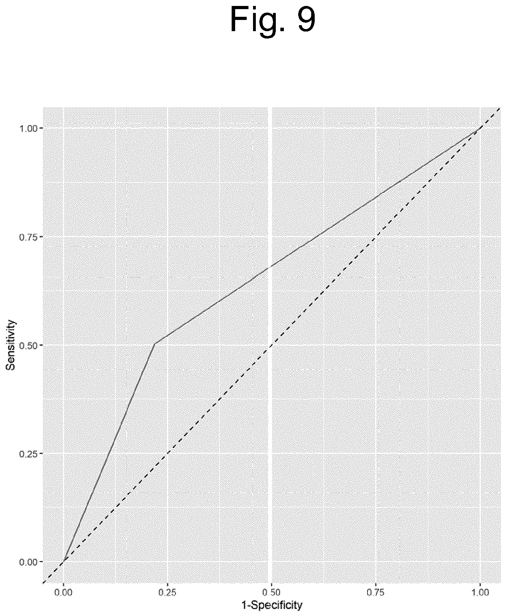

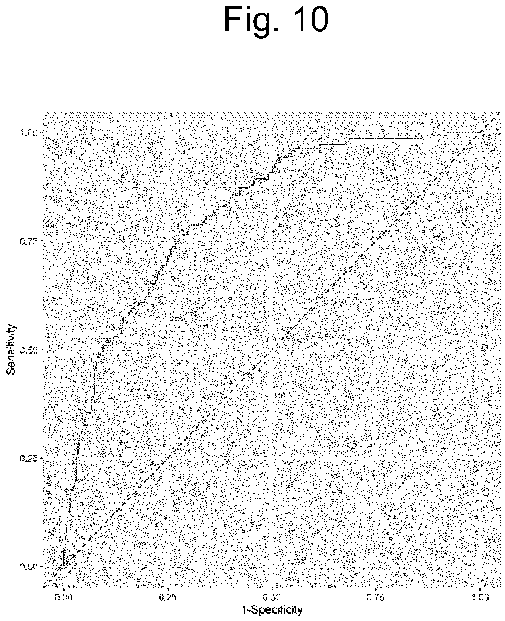

Second, the correlation of the RoPro with OS survival was also replicated in the phase III study OAK (n=1,187, $P$=8.38x10$^{-60}$, r2=0.20, C-index=0.69). Patients with RoPro>0.82 (cut-off equal to 90%-quantile in Flatiron Health for dedicated advanced NSCLC RoPro, n=34) had poorer OS prognosis (HR 3.65; 95% CI 2.59-5.12). Area under the curve values again outperformed RMHS (Figures 9 and 10).

In the phase I study, the inventors saw a correlation of high RoPro (>1.18, see definition above, n=9) with study dropout. All high RoPro patients left the study very early, either due to death (n=5) or progressive disease (n=4). Only one patient could receive more than one treatment cycle, although all patients had an ECOG performance status value of 1 at baseline (a study inclusion criterion). Typically, study protocols require a life expectancy of >12 weeks for enrollment since drug efficacy is potentially masked in patients with a particularly bad prognosis. However, the RoPro analysis demonstrates that with current practice it is not always possible to identify all high-risk patients a priori. With the RoPro available, however, respective information can retrospectively be included to exclude patients from analysis or to perform stratified analysis.

In the phase III study, the inventors assessed potential impact of using the prognostic score as a covariate parameter in comparison of OS between the treatment arms. In plain analysis, an HR of 0.794 (95% CI 0.690-0.913, $P$=0.0012) was observed for atezolizumab versus docetaxel, in accordance with published results (Connolly, J. G., Schneeweiss, S., Glynn, R. J. & Gagne, J. J. Quantifying bias reduction with fixed-duration versus all-available covariate assessment periods. Pharmacoepidemiol. Drug Saf. 28, 665-670 (2019)). Although not required in a randomized study, the inventors adjusted the efficacy analysis for baseline RoPro. The inventors could confirm the efficacy gain with atezolizumab and obtained a HR of 0.760 (95% CI 0.666-0.875, P=0.00013) in the RoPro adjusted analysis.

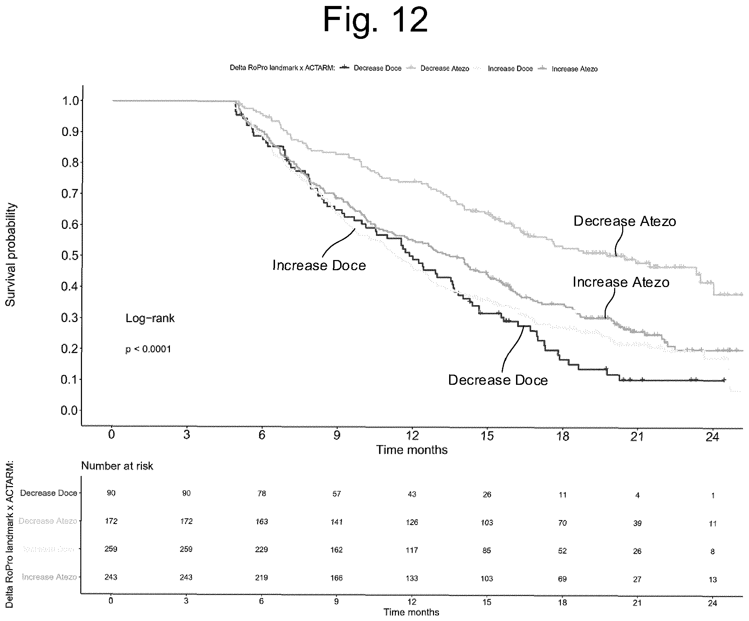

Finally, the inventors made an initial assessment whether changes of RoPro over time might be indicative of later events. An ad hoc visualization is given in Figure 11. For patients who died (upper curve), the score worsened markedly during the last 100 days prior to the event. A less pronounced increase was seen towards progression (middle curve) while partial (n=191) and complete (n=11) responders did not show an increase (lower curve). To formalize this observation, the inventors the inventors performed a landmark analysis as described in Houwelingen, H. C. V. Dynamic Prediction by Landmarking in Event History Analysis. Scand. J. Stat. 34, 70-85 (2007). The landmark was set at 145 days until where 75% of the study participants survived. Those patients were classified into two groups, defined by either RoPro increase or decrease from baseline to the landmark. In Figure 12 survival curves after the landmark shown patients are shown. Patients with increasing RoPro until the landmark (yellow) have significantly shorter survival time after the landmark than patients with early decrease (blue) of the RoPro, HR of 1.90 (95% CI 1.57-2.31, $P$=8.1x10$^{-11}$).

*Discussion*

In this analysis of 122,694 patients from 17 cancer cohorts, 27 independent OS risk factors were identified and used to define a prognostic model, the RoPro. It showed a previously unseen level of correlation with OS. Validation analyses using data from two independent clinical studies confirmed these results. To the inventors' knowledge, the RoPro is based on the largest dataset used for prognostic modelling to date, overcoming typical limitations. Furthermore, the inventors have proven within a classic statistical framework that a multitude of prognostic factors have an independent contribution to risk modelling, after adjustment for correlation between the risk factors. The results were highly consistent when using different modeling approaches (backward-selection and regularized regression).

The RoPro may comprise 27 clinical parameters combined into a single score and can be readily applied to new data sets. A substantial improvement in performance over alternative scores from models such as the RMHS, the IPI or the IMDC has been demonstrated.

By construction, all RoPro variables are typically available in routine clinical practice in the U.S. The majority of variables are also available in clinical practice in other countries and clinical studies conducted by pharmaceutical industry. Patient-specific missingness of five to ten variables is tolerated for computing a patient's RoPro.

Finally, the RoPro can be applied across cancer indications other than the 17 used to build the model, as demonstrated by the performance in BP29428, where 40% of patients had cancer indications not available in Flatiron Health.

The inventors' analysis in study BP29428 (emactuzumab/atezolizumab combination, NCT02323191) showed that RoPro correlated not only with OS but also with particular early study dropout. Hence, using RoPro to exclude very high-risk patients may help protect patients from unnecessary exposure to study procedural burden and potential adverse events, although this needs to be balanced against the benefits of allowing such patients access to potentially effective novel drugs.

Landmark analysis in the OAK study demonstrated that an early increase/decrease of the RoPro is indicative of later survival events. While biologically plausible, we note that this observation is not self-evident. A possible alternative scenario would have been one of early-stage and late-stage prognostic factors, where early-stage factors do not deteriorate from a certain time point onwards and late-stage mechanisms are indicative of developments very close to death event.

The increase of the RoPro towards death is a feature that generates possible applications of high potential. First, during dose escalation in first-in-human studies it is plausible hypothesis that the RoPro will increase under inefficient drug dosages or treatments, but will stay stable or even improve under effective treatment. In this way, group level analysis of RoPro time course by treatment arm or drug dosage can give valuable insights on potential drug efficacy. Increase of RoPro might, in combination with death events, be used as a surrogate end-point in clinical studies.

Continuous monitoring of the RoPro over time could accompany treatment decision making. The inventors' analysis of OAK study data indicated that baseline high-risk patients whose scores in addition worsen over time are unlikely to benefit from therapy, the majority of such patients died within very short time. Therefore, patients with increasing RoPro could be switched to another treatment. Observing stable scores or even slightly improving scores might indicate treatment benefits and could be used, along with many other considerations, in decisions about continuing treatment.

The fact that RoPro works well on "new" cancer indications as exemplified by its application to the Phase I study, which recruited many patients from outside the US, increases confidence that the RoPro is robust across geographic regions and ethnicities.

While uncertainties and inaccuracies are expected to be encountered with retrospective real-world data analysis, the inventors' findings suggest that the power gain obtained by sample size overcomes potential bias.

*Further Performance Evaluation, including evaluation of RoProl6*

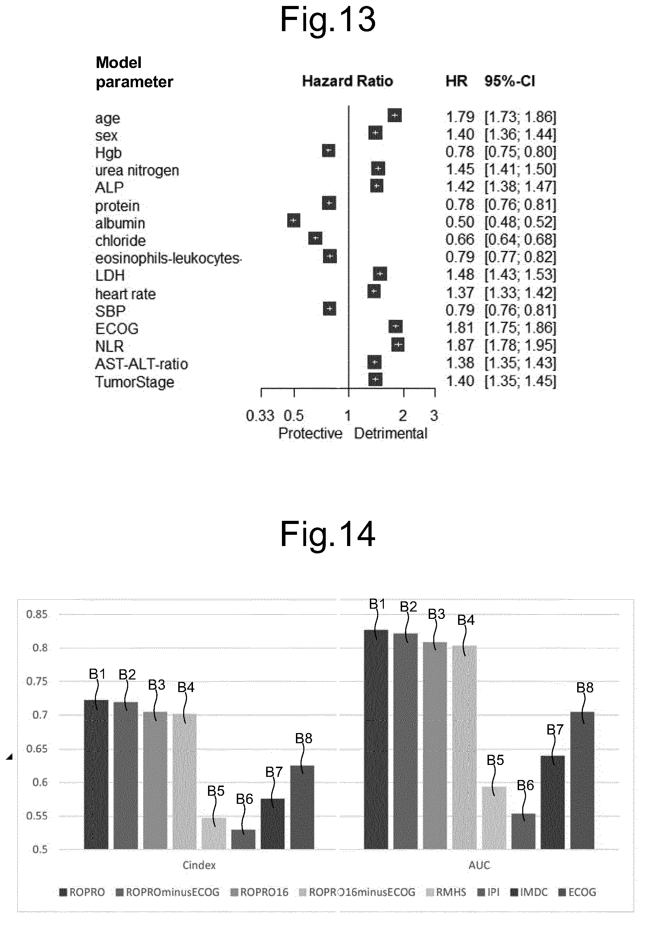

Figures 13-18 depict results from further example implementations of models of the present disclosure, including models using 27 parameters (ROPRO), models using 16 parameters (ROPRO16), and variations on these two models that omit the Eastern cooperative oncology group (ECOG) performance status parameter (ROPROminusECOG and ROPRO16minusECOG).

The results make comparisons with prior art alternative prognostic scores, including RMHS, IPI, IMDC, and ECOG (all mentioned above).

Figure 13 is a graph corresponding to the graph of Figure 3 except using the ROPRO16 model rather than the full ROPRO model with 27 parameters. The parameter weightings shown in Figure 13, which are derived from the HR values as described above, are seen to be broadly similar to the weightings for the corresponding parameters in Figure 3.

Figure 14 is bar chart comparing performance between ROPRO (labelled B1), ROPROminusECOG (labelled B2), ROPRO16 (labelled B3), and ROPRO16minusECOG (labelled B4) and the prior art prognostic scores RMHS (labelled B5), IPI (labelled B6), IMDC (labelled B7), and ECOG (labelled B8). Two comparisons are shown: Cindex (comparing general performance for OS prediction) and AUC (comparing performance to predict 12 week survival). As can be seen, all of the models of the present disclosure, even when reduced to using 16 or 15 parameters outperform all of the prior art prognostic scores considered, for both Cindex and AUC. The relatively small difference between using 15 parameters and 16 parameters demonstrates the relative robustness of the approach to missingness in the data even where the model is in the reduced (16 parameter) form.

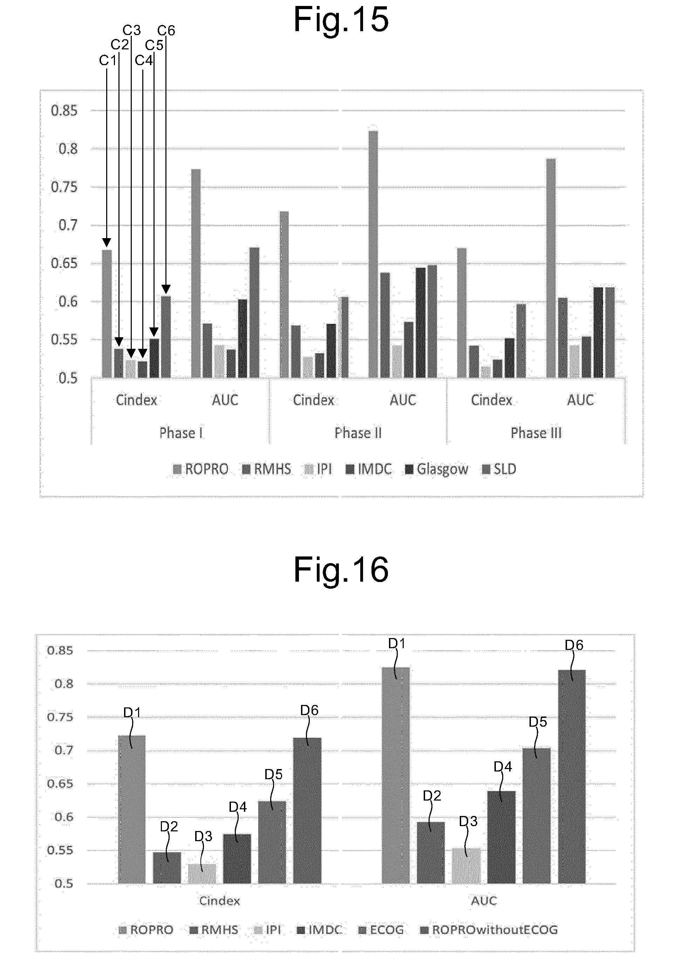

Figure 15 is a bar chart comparing performance between ROPRO (labelled C1 in the left-most sub-chart) and prior art alternative prognostic scores RMHS (labelled C2 in the left-most sub-chart), IPI (labelled C3 in the left-most sub-chart), IMDC (labelled C4 in the left-most sub-chart), Glasgow (referred to above and labelled C5 in the left-most sub-chart) and SLD (which stands for the sum of the longest diameters of tumours and is labelled C6 in the left-most sub-chart). The bars in the five other sub-charts follow the same sequence of C1-C6 although these labels are omitted for clarity. The database used in this demonstration covered 20 clinical studies from phase 1 to phase 3 across different cancer indications. The bar chart shows that ROPRO outperforms the other scores for both Cindex and AUC in phase 1, 2 and 3.

Figure 16 is a bar chart comparing performance between ROPRO (labelled D1) and ROPROwithoutECOG (which is the same as ROPROminusECOG referred to above, and is labelled D6) and prior art alternative prognostic scores RMHS (labelled D2), IPI (labelled D3), IMDC (labelled D4) and ECOG (labelled D5). The database used in this demonstration covered 20 clinical studies from phase 1 to phase 3 across different cancer indications. The bar chart shows again that both models of the present disclosure outperform all of the alternative prognostic scores considered.

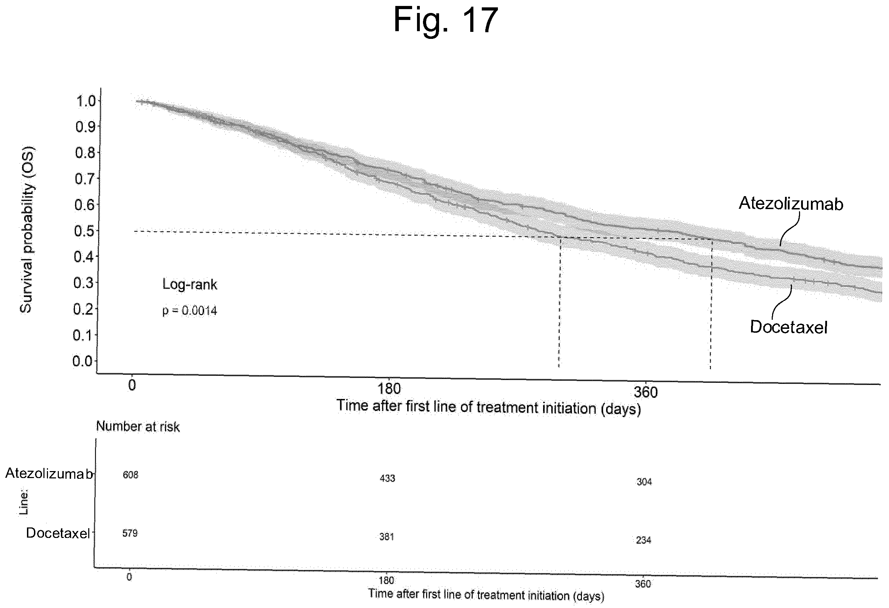

Figure 17 is a graph depicting a Kaplan-Meyer Survival Analysis of OAK study. The study showed that Atezolizumab treatment results in a statistically significant and clinically relevant improvement in OS vs Docetaxel in 2L/3L NSCLC. This survival analysis confirms the finding.

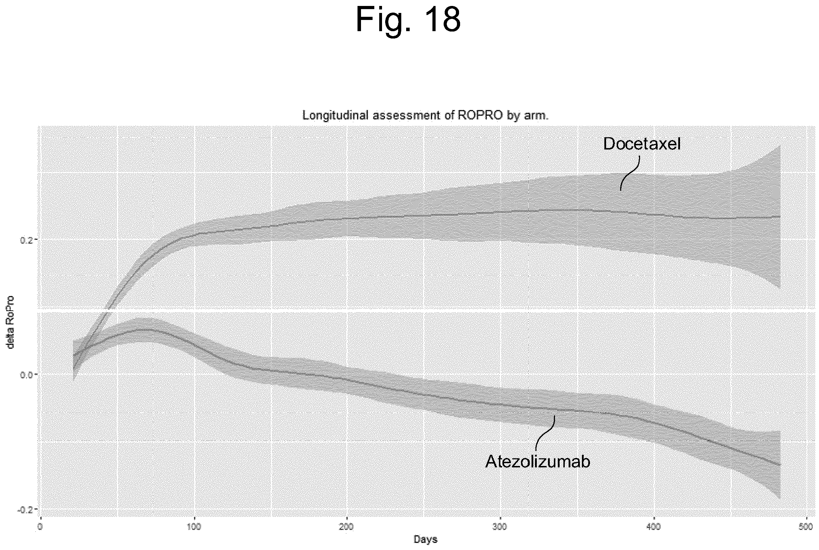

Figure 18 is a graph depicting a delta ROPRO analysis of the two arms of the study referred to in relation to Figure 17 above. The ROPRO score is significantly increasing in patients in the Docetaxel group compared to Atezolizumab group indicating that the delta ROPRO is indicative of treatment efficacy. This trend is in addition seen earlier than in the survival analysis.

**Claims**

1. A computer-implemented method of determining a risk of mortality of a cancer patient, the method comprising:

   receiving patient data representing information about a cancer patient;
   using a mathematical model of mortality risk to determine a risk of mortality of the cancer patient based on the received patient data, wherein the determination of the risk of mortality comprises calculating a numerical score representing the risk of mortality; and

   **characterized in that** the method further comprises:
   outputting the determined risk of mortality, wherein the outputting of the determined risk of mortality comprises outputting the calculated numerical score, wherein:
   the mathematical model models a relationship determined from training data between the risk of mortality and values of at least the following model parameters:

   (i) age;
   (ii) gender;
   (iii) haemoglobin or haematocrit level in blood;
   (iv) urea nitrogen level in serum or plasma;
   (v) alkaline phosphatase enzymatic activity level in serum or plasma;
   (vi) protein level in serum or plasma;
   (vii) level of albumin in serum or plasma;
   (viii) chloride or sodium level in serum or plasma;
   (ix) ratio of eosinophils to leukocytes in blood;
   (x) lactate dehydrogenase enzymatic activity level in serum or plasma;
   (xi) heart rate;
   (xii) systolic blood pressure;
   (xiii) Eastern cooperative oncology group (ECOG) performance status;
   (xiv) ratio of neutrophils to lymphocytes in blood;
   (xv) ratio of aspartate aminotransferase enzymatic activity level in serum or plasma to alanine aminotransferase enzymatic activity level in serum or plasma; and
   (xvi) TNM classification of tumor stage.

2. The method of claim 1, wherein the mathematical model models the relationship determined from training data between the risk of mortality and values of the model parameters (i)-(xvi) and at least the following parameters:

   (xvii) smoking history;
   (xviii) number of metastatic sites;
   (xix) platelet level in blood;
   (xx) calcium level in serum or plasma;
   (xxi) glucose level in blood;
   (xxii) ratio of lymphocytes to leukocytes in blood;
   (xxiii) level of bilirubin in serum or plasma;
   (xxiv) level of monocytes in blood;
   (xxv) level of oxygen saturation in arterial blood; and
   (xxvi) body mass index.

3. The method of claim 2, wherein the mathematical model models the relationship determined from training data between the risk of mortality and values of the model parameters (i)-(xxvi) and at least the following parameter:
   (xxvii) alanine aminotransferase enzymatic activity level in serum or plasma.

4. The method of claim 3, wherein the mathematical model models the relationship determined from training data between the risk of mortality and values of the model parameters (i)-(xxvii) and at least the following parameters:

   (xxviii) level of eosinophils in blood; and
   (xxix) diastolic blood pressure.

5. The method of any preceding claim, wherein the determination of the mortality risk further comprises comparing the calculated score to one or more predetermined threshold values, or to calculated scores for other cancer patients.

6. The method of any preceding claim, wherein the model comprises a weighted sum of deviations in the patient data from mean values of the model parameters in the training data, and the model is formed by determining the weightings from the training data.

7. The method of any preceding claim, wherein the model is formed by performing multivariable Cox regression analysis on the training data for a plurality of subjects, preferably at least 1000 subjects.

8. The method of any preceding claim, wherein:

   the model is formed by: assigning a respective weighting, $w_i$, to each of the model parameters, and determining a respective mean, $m_i$, of values of each model parameter over the training data; and
   the determination of the mortality risk comprises calculating a numerical score according to the following formula:

$$\text{score} = \sum_i w_i(m_{ij} - m_i)$$

   where $w_i$ is the weighting of the $i$-th model parameter, $m_i$ is the mean of the $i$-th model parameter, and $m_{ij}$ is the value of the $i$-th model parameter for a $j$-th cancer patient for whom the score is to be calculated.

9. A method of assessing an anti-cancer therapy, comprising determining a risk of mortality of a patient at plural different times while the patient is receiving the anti-cancer therapy by performing the method of any preceding claim at each of the plural times, and analysing the resulting determined risks to determine an efficacy of the anti-cancer therapy.

10. A method of selecting cancer patients for treatment with an anti-cancer therapy, comprising determining a risk of mortality of a candidate patient using the method of any of claims 1-8 and using the determined risk to decide whether to select each candidate patient.

11. A computer program comprising instructions which, when the program is executed by a computer, cause the computer to carry out the method of any preceding claim.

**Patentansprüche**

1. Computerimplementiertes Verfahren zur Bestimmung des Sterblichkeitsrisikos eines Krebspatienten, wobei das Verfahren Folgendes umfasst:

   Empfangen von Patientendaten, die Informationen über einen Krebspatienten darstellen;
   Verwenden eines mathematischen Modells des Sterblichkeitsrisikos, um ein Sterblichkeitsrisiko des Krebspatienten basierend auf den empfangenen Patientendaten zu bestimmen, wobei die Bestimmung des Sterblichkeitsrisikos das Berechnen eines numerischen Scores umfasst, der das Sterblichkeitsrisiko darstellt; und
   **dadurch gekennzeichnet, dass** das Verfahren ferner Folgendes umfasst:
   Ausgeben des bestimmten Sterblichkeitsrisikos, wobei das Ausgeben des bestimmten Sterblichkeitsrisikos das Ausgeben des berechneten numerischen Scores umfasst, wobei:
   das mathematische Modell eine Beziehung, die aus Trainingsdaten bestimmt wird, zwischen dem Sterblichkeitsrisiko und Werten von mindestens den folgenden Modellparametern modelliert:

   (i) Alter;
   (ii) Geschlecht;

(iii) Hämoglobin- oder Hämatokritspiegel im Blut;

(iv) HarnstoffStickstoff im Serum oder Plasma;

(v) Enzymaktivitätsniveau von alkalischer Phosphatase im Serum oder Plasma;

(vi) Proteinspiegel im Serum oder Plasma;

(vii) Albuminspiegel im Serum oder Plasma;

(viii) Chlorid- oder Natriumspiegel im Serum oder Plasma;

(ix) Verhältnis von Eosinophilen zu Leukozyten im Blut;

(x) Enzymaktivitätsniveau von Lactatdehydrogenase im Serum oder Plasma;

(xi) Herzfrequenz;

(xii) systolischer Blutdruck;

(xiii) Leistungsstatus der Eastern Cooperative Oncology Group (ECOG);

(xiv) Verhältnis von Neutrophilen zu Lymphozyten im Blut;

(xv) Verhältnis des Enzymaktivitätsniveaus von Aspartataminotransferase im Serum oder Plasma zum Enzymaktivitätsniveau von Alaninaminotransferase im Serum oder Plasma; und

(xvi) TNM-Klassifizierung des Tumorstadiums.

2. Verfahren nach Anspruch 1, wobei das mathematische Modell die Beziehung, die aus Trainingsdaten bestimmt wird, zwischen dem Sterblichkeitsrisiko und Werten der Modellparameter (i)-(xvi) und mindestens den folgenden Parametern modelliert:

(xvii) Raucherhistorie;

(xviii) Anzahl von Metastasierungsstellen;

(xix) Blutplättchenspiegel im Blut;

(xx) Calciumspiegel im Serum oder Plasma;

(xxi) Glucosespiegel im Blut;

(xxii) Verhältnis von Lymphozyten zu Leukozyten im Blut;

(xxiii) Bilirubinspiegel im Serum oder Plasma;

(xxiv) Monozytenzahl im Blut;

(xxv) Sauerstoffsättigungsgrad im arteriellen Blut; und

(xxvi) Körpermasseindex.

3. Verfahren nach Anspruch 2, wobei das mathematische Modell die Beziehung, die aus Trainingsdaten bestimmt wird, zwischen dem Sterblichkeitsrisiko und Werten der Modellparameter (i)-(xxvi) und mindestens dem folgenden Parameter modelliert:

(xxvii) Enzymaktivitätsniveau von Alaninaminotransferase im Serum oder Plasma.

4. Verfahren nach Anspruch 3, wobei das mathematische Modell die Beziehung, die aus Trainingsdaten bestimmt wird, zwischen dem Sterblichkeitsrisiko und Werten der Modellparameter (i)-(xxvii) und mindestens den folgenden Parametern modelliert:

(xxviii) Eosinophilenzahl im Blut; und

(xxix) diastolischer Blutdruck.

5. Verfahren nach einem der vorhergehenden Ansprüche, wobei die Bestimmung des Sterblichkeitsrisikos ferner das Vergleichen des berechneten Scores mit einem oder mehreren vorbestimmten Schwellenwerten oder mit berechneten Scores für andere Krebspatienten umfasst.

6. Verfahren nach einem der vorhergehenden Ansprüche, wobei das Modell eine gewichtete Summe von Abweichungen in den Patientendaten von Mittelwerten der Modellparameter in den Trainingsdaten umfasst und das Modell durch Bestimmen der Gewichtungen aus den Trainingsdaten gebildet wird.

7. Verfahren nach einem der vorhergehenden Ansprüche, wobei das Modell durch Durchführen einer multivariablen Cox-Regressionsanalyse an den Trainingsdaten für eine Vielzahl von Probanden, vorzugsweise mindestens 1000 Probanden, gebildet wird.

8. Verfahren nach einem der vorhergehenden Ansprüche, wobei:

das Modell durch Folgendes gebildet wird: Zuweisen einer jeweiligen Gewichtung $w_i$ zu jedem der Modellpara-

meter und Bestimmen eines jeweiligen Mittelwerts $m_i$ von Werten jedes Modellparameters über die Trainingsdaten; und

die Bestimmung des Sterblichkeitsrisikos das Berechnen eines numerischen Scores gemäß der folgenden Formel umfasst:

$$\text{Score} = \sum_i w_i\,(m_{ij} - m_i)$$

wobei $w_i$ die Gewichtung des $i$-ten Modellparameters ist, $m_i$ der Mittelwert des $i$-ten Modellparameters ist und $m_{ij}$ der Wert des $i$-ten Modellparameters für einen $j$-ten Krebspatienten ist, für den der Score berechnet werden soll.

9. Verfahren zur Beurteilung einer Antikrebstherapie, umfassend das Bestimmen eines Sterblichkeitsrisikos eines Patienten zu mehreren verschiedenen Zeitpunkten, während der Patient die Antikrebstherapie erhält, durch Durchführen des Verfahrens nach einem der vorhergehenden Ansprüche zu jedem der mehreren Zeitpunkte und Analysieren der resultierenden bestimmten Risiken, um eine Wirksamkeit der Antikrebstherapie zu bestimmen.

10. Verfahren zur Auswahl von Krebspatienten zur Behandlung mit einer Antikrebstherapie, umfassend das Bestimmen eines Sterblichkeitsrisikos eines Patientenkandidats unter Verwendung des Verfahrens nach einem der Ansprüche 1-8 und Verwenden des bestimmten Risikos, um zu entscheiden, ob jeder Patientenkandidat ausgewählt werden soll.

11. Computerprogramm, umfassend Anweisungen, die, wenn das Programm von einem Computer ausgeführt wird, den Computer veranlassen, das Verfahren nach einem der vorhergehenden Ansprüche auszuführen.

**Revendications**

1. Procédé mis en œuvre par ordinateur de détermination du risque de mortalité d'un patient atteint de cancer, le procédé comprenant :

la réception de données patient représentant des informations au sujet d'un patient atteint de cancer ;
l'utilisation d'un modèle mathématique de risque de mortalité pour déterminer un risque de mortalité du patient atteint de cancer en se basant sur les données patient reçues, la détermination du risque de mortalité comprenant le calcul d'un score numérique représentant le risque de mortalité ; et **caractérisé en ce que** le procédé comprend en outre :
la sortie du risque de mortalité déterminé, la sortie du risque de mortalité déterminé comprenant la sortie du score numérique calculé, dans lequel :
le modèle mathématique modélise une relation déterminée à partir de données d'entraînement entre le risque de mortalité et les valeurs d'au moins les paramètres du modèle suivants :

(i) âge ;
(ii) sexe ;
(iii) taux d'hémoglobine ou d'hématocrite dans le sang ;
(iv) taux d'azote uréique dans le sérum ou le plasma ;
(v) taux d'activité enzymatique de l'alcaline phosphatase dans le sérum ou le plasma ;
(vi) taux de protéines dans le sérum ou le plasma ;
(vii) taux d'albumine dans le sérum ou le plasma ;
(viii) taux de chlorure ou de sodium dans le sérum ou le plasma ;
(ix) rapport des éosinophiles aux leucocytes dans le sang ;
(x) taux d'activité enzymatique de la lactate déshydrogénase dans le sérum ou le plasma ;
(xi) rythme cardiaque ;
(xii) pression systolique ;
(xiii) score de performance selon l'Eastern Cooperative Oncology Group (ECOG) ;
(xiv) rapport des neutrophiles aux lymphocytes dans le sang ;
(xv) rapport du taux d'activité enzymatique de l'aspartate aminotransférase dans le sérum ou le plasma au taux d'activité enzymatique de l'alanine aminotransférase dans le sérum ou le plasma ; et
(xvi) classification TNM du stade tumoral.

**2.** Procédé selon la revendication 1, dans lequel le modèle mathématique modélise la relation déterminée à partir des données d'entraînement entre le risque de mortalité et les valeurs des paramètres du modèle (i) à (xvi) et au moins les paramètres suivants :

(xvii) antécédents de tabagisme ;
(xviii) nombre de sites métastatiques ;
(xix) taux de plaquettes dans le sang ;
(xx) taux de calcium dans le sérum ou le plasma ;
(xxi) glycémie sanguine ;
(xxii) rapport des lymphocytes aux leucocytes dans le sang ;
(xxiii) taux de bilirubine dans le sérum ou le plasma ;
(xxiv) taux de monocytes dans le sang ;
(xxv) taux de saturation en oxygène dans le sang artériel ; et
(xxvi) indice de masse corporelle.

**3.** Procédé selon la revendication 2, dans lequel le modèle mathématique modélise la relation déterminée à partir des données d'entraînement entre le risque de mortalité et les valeurs des paramètres du modèle (i) à (xxvi) et au moins le paramètre suivant :
(xxvii) taux d'activité enzymatique de l'alanine aminotransférase dans le sérum ou le plasma.

**4.** Procédé selon la revendication 3, dans lequel le modèle mathématique modélise la relation déterminée à partir des données d'entraînement entre le risque de mortalité et les valeurs des paramètres du modèle (i) à (xxvii) et au moins les paramètres suivants :

(xxviii) taux d'éosinophiles dans le sang ; et
(xxix) pression diastolique.

**5.** Procédé selon une quelconque revendication précédente, dans lequel la détermination du risque de mortalité comprend en outre la comparaison du score calculé à une ou plusieurs valeurs seuil prédéterminées, ou à des scores calculés pour d'autres patients atteints de cancer.

**6.** Procédé selon une quelconque revendication précédente, dans lequel le modèle comprend une somme pondérée d'écarts dans les données patient à partir des valeurs moyennes des paramètres du modèle dans les données d'entraînement, et le modèle est formé en déterminant les pondérations à partir des données d'entraînement.

**7.** Procédé selon une quelconque revendication précédente, dans lequel le modèle est formé en réalisant une analyse par régression de Cox multivariable sur les données d'entraînement pour une pluralité de sujets, de préférence au moins 1000 sujets.

**8.** Procédé selon une quelconque revendication précédente, dans lequel :

le modèle est formé en : attribuant une pondération respective, $w_i$, à chacun des paramètres du modèle, et déterminant une moyenne respective, $m_i$, des valeurs de chaque paramètre du modèle sur les données d'entraînement ; et
la détermination du risque de mortalité comprend le calcul d'un score numérique conformément à la formule suivante :

$$score = \sum w_i (m_{ij} - m_i)$$

où $w_i$ représente la pondération du ième paramètre du modèle, $m_i$ représente la moyenne du ième paramètre du modèle, et $m_{ij}$ représente la valeur du ième paramètre du modèle pour un jème patient atteint de cancer pour lequel le score doit être calculé.

**9.** Procédé d'évaluation d'une thérapie anticancéreuse, comprenant la détermination du risque de mortalité d'un patient à plusieurs temps différents alors que le patient reçoit la thérapie anticancéreuse en effectuant le procédé selon une quelconque revendication précédente à chacun des plusieurs temps, et l'analyse des risques déterminés résultants pour déterminer une efficacité de la thérapie anticancéreuse.

10. Procédé de sélection de patients atteints de cancer en vue d'un traitement par une thérapie anticancéreuse, comprenant la détermination du risque de mortalité d'un patient candidat en utilisant le procédé selon l'une quelconque des revendications 1 à 8 et en utilisant le risque déterminé pour décider s'il faut sélectionner chaque patient candidat.

11. Programme informatique comprenant des instructions qui, lorsque le programme est exécuté par un ordinateur, amènent l'ordinateur à réaliser le procédé selon une quelconque revendication précédente.

# Fig. 1

S1 — Receive patient data

S2 — Determine risk (score)

S3 — Output risk (score)

# Fig. 2

# Fig. 3

| Parameter | Hazard Ratio | HR | 95% CI |
|---|---|---|---|
| **Host** | | | |
| albumin | | 0.44 | 0.42–0.46 |
| chloride | | 0.67 | 0.65–0.70 |
| Hgb | | 0.73 | 0.70–0.75 |
| lymphocytes–leukocytes–ratio | | 0.75 | 0.67–0.83 |
| platelets | | 0.78 | 0.75–0.80 |
| protein | | 0.78 | 0.75–0.81 |
| SBP | | 0.80 | 0.77–0.82 |
| eosinophils–leukocytes–ratio | | 0.82 | 0.79–0.84 |
| oxygen | | 0.86 | 0.83–0.88 |
| ALT | | 0.87 | 0.83–0.92 |
| glucose | | 1.06 | 1.03–1.10 |
| bilirubin | | 1.20 | 1.16–1.24 |
| AST–ALT–ratio | | 1.26 | 1.21–1.31 |
| monocytes | | 1.29 | 1.24–1.34 |
| calcium | | 1.31 | 1.27–1.36 |
| sex | | 1.33 | 1.28–1.37 |
| heart rate | | 1.38 | 1.33–1.42 |
| LDH | | 1.39 | 1.34–1.45 |
| urea nitrogen | | 1.40 | 1.35–1.45 |
| NLR | | 1.46 | 1.28–1.66 |
| ALP | | 1.47 | 1.42–1.53 |
| age | | 1.52 | 1.46–1.57 |
| ECOG | | 1.71 | 1.66–1.77 |
| **Lifestyle** | | | |
| BMI | | 0.84 | 0.82–0.87 |
| smoking | | 1.31 | 1.26–1.37 |
| **Tumor** | | | |
| Number of metastatic sites | | 1.16 | 1.13–1.20 |
| TumorStage | | 1.42 | 1.36–1.47 |

0.4    0.75 1    1.5

Protective  Detrimental

22

# Fig. 4

RMHS: —— low —— high

**Number at risk**

| RMHS: | 0 | 180 | 360 | 540 | 720 | 900 | 1080 | 1260 | 1440 |
|---|---|---|---|---|---|---|---|---|---|
| low | 112355 | 81917 | 58864 | 43723 | 33454 | 25661 | 19905 | 15391 | 11776 |
| high | 10339 | 5072 | 3007 | 1979 | 1380 | 1006 | 772 | 604 | 455 |

Time after first line of treatment initiation (days)

# Fig. 5

RoPro class: —— remaining 90% —— upper 10%

**Number at risk**

| RoPro class: | 0 | 180 | 360 | 540 | 720 | 900 | 1080 | 1260 | 1440 |
|---|---|---|---|---|---|---|---|---|---|
| remaining 90% | 110424 | 83238 | 60315 | 44935 | 34408 | 26415 | 20520 | 15894 | 12166 |
| upper 10% | 12270 | 3751 | 1556 | 767 | 426 | 252 | 157 | 101 | 65 |

Time after first line of treatment initiation (days)

# Fig. 6

RoPro decile: decile 1, decile 3, decile 5, decile 7, decile 9, decile 2, decile 4, decile 6, decile 8, decile 10

Survival probability vs Time after first line of treatment initiation (days)

## Number at risk

| RoPro decile | 0 | 180 | 360 | 540 | 720 | 900 | 1080 | 1260 | 1440 |
|---|---|---|---|---|---|---|---|---|---|
| decile 1 | 12270 | 10885 | 9629 | 8502 | 7490 | 6467 | 5543 | 4721 | 3964 |
| decile 2 | 12269 | 10600 | 8984 | 7481 | 6204 | 5027 | 4084 | 3212 | 2472 |
| decile 3 | 12269 | 10379 | 8321 | 6648 | 5227 | 4027 | 3089 | 2382 | 1775 |
| decile 4 | 12270 | 10137 | 7810 | 5831 | 4363 | 3215 | 2376 | 1731 | 1299 |
| decile 5 | 12269 | 9752 | 7029 | 4985 | 3603 | 2599 | 1910 | 1376 | 980 |
| decile 6 | 12269 | 9309 | 6313 | 4177 | 2882 | 1996 | 1434 | 1015 | 691 |
| decile 7 | 12270 | 8512 | 5151 | 3247 | 2158 | 1457 | 1006 | 688 | 478 |
| decile 8 | 12269 | 7495 | 4131 | 2451 | 1535 | 1025 | 686 | 491 | 322 |
| decile 9 | 12269 | 6169 | 2947 | 1613 | 946 | 602 | 392 | 278 | 185 |
| decile 10 | 12270 | 3751 | 1556 | 767 | 426 | 252 | 157 | 101 | 65 |

Time after first line of treatment initiation (days)

# Fig. 7

# Fig. 8

# Fig. 9

# Fig. 10

# Fig. 11

# Fig. 12

# Fig.13

# Fig.14

# Fig.15

# Fig.16

# Fig. 17

EP 4 158 655 B1

Fig. 18

Longitudinal assessment of ROPRO by arm.

## REFERENCES CITED IN THE DESCRIPTION

**Non-patent literature cited in the description**

- **BANKS, E. et al.** Erectile dysfunction severity as a risk marker for cardiovascular disease hospitalisation and all-cause mortality: a prospective cohort study. *PLoS Med.*, 2013, vol. 10, e1001372
- **HU, F. B. et al.** Adiposity as compared with physical activity in predicting mortality among women. *N. Engl. J. Med.*, 2004, vol. 351, 2694-2703
- **MCGEE, D. L.** ; **LIAO, Y.** ; **CAO, G.** ; **COOPER, R. S.** Self-reported health status and mortality in a multiethnic US cohort.. *Am. J. Epidemiol.*, 1999, vol. 149, 41-46
- **THUN, M. J. et al.** Alcohol consumption and mortality among middle-aged and elderly U.S. adults. *N. Engl. J. Med.*, 1997, vol. 337, 1705-1714
- **TOTA-MAHARAJ, R. et al.** Coronary artery calcium for the prediction of mortality in young adults <45 years old and elderly adults >75 years old.. *Eur. Heart J.*, 2012, vol. 33, 2955-2962
- **NIEDER, C.** ; **DALHAUG, A.** A new prognostic score derived from phase I study participants with advanced solid tumours is also valid in patients with brain metastasis.. *Anticancer Res.*, 2010, 977-9
- International Non-Hodgkin's Lymphoma Prognostic Factors Project. A predictive model for aggressive non-Hodgkin's lymphoma.. *N. Engl. J. Med.*, 1993, vol. 329, 987-994
- **KO, J. et al.** The International Metastatic Renal Cell Carcinoma Database Consortium model as a prognostic tool in patients with metastatic renal cell carcinoma previously treated with first-line targeted therapy: a population-based study. *Lancet Oncol.*, 2015, vol. 16, 293-300
- **KINOSHITA, A. et al.** The Glasgow Prognostic Score, an inflammation based prognostic score, predicts survival in patients with hepatocellular carcinoma. *BMC Cancer*, 2013, vol. 52
- **SUDLOW, C. et al.** UK Biobank: An Open Access Resource for Identifying the Causes of a Wide Range of Complex Diseases of Middle and Old Age. *PLoS Med.*, 2015, vol. 12, e1001779
- **GANNA, A.** ; **INGELSSON, E.** 5-year mortality predictors in 498 103 UK Biobank participants: a prospective population-based study.. *The Lancet*, 2015, vol. 386, 533-540
- **CHARLSON, M. E.** ; **POMPEI, P.** ; **ALES, K. L.** ; **C.RONALD, M.** A new method of classifying prognostic comorbidity in longitudinal studies: Development and validation.. *J Chronic Dis.*, 1987, 373-383
- History of cancer and mortality in community-dwelling older adults. **ELIZABETH KVALE et al.** CANCER EPIDEMIOLOGY. ELSEVIER, 13 July 2010, vol. 35, 30-36
- **COX DR.** Regression Models and Life-Tables.. *Journal of the Royal Statistical Society. Series B (Methodological)*, vol. 34
- **STOCK, C.** ; **MONS, U.** ; **BRENNER, H.** Projection of cancer incidence rates and case numbers until 2030: A probabilistic approach applied to German cancer registry data (1999-2013). *Cancer Epidemiol*, 2018, vol. 57, 110-119
- **CONNOLLY, J. G.** ; **SCHNEEWEISS, S.** ; **GLYNN, R. J.** ; **GAGNE, J. J.** Quantifying bias reduction with fixed-duration versus all-available covariate assessment periods. *Pharmacoepidemiol. Drug Saf.*, 2019, vol. 28, 665-670
- The statistical analysis of failure time data. **KALBFLEISCH, JD** ; **PRENTICE, RL.** Wiley Series in Probability and Statistics. 2002
- On Ranking in Survival Analysis: Bounds on the Concordance Index. **STECK, H.** ; **KRISHNAPURAM, B.** ; **DEHING-OBERIJE, C.** ; **LAMBIN, P.** ; **RAYKAR, V. C.** Advances in Neural Information Processing Systems. Curran Associates, Inc., 2008, vol. 20, 1209-1216
- R: A language and environment for statistical computing. *R Foundation for Statistical Computing*, 2013, http://www.R-project.org
- **SIMON N** ; **FRIEDMAN J** ; **HASTIE T** ; **TIBSHIRANI R.** Regularization Paths for Cox's Proportional Hazards Model via Coordinate Descent.. *J. Stat. Softw. Artic.*, 2011, vol. 39 (5), 1-13
- **RILEY, R. D. et al.** Minimum sample size for developing a multivariable prediction model: PART II - binary and time-to-event outcomes. *Stat. Med.*, 2019, vol. 38, 1276-1296
- **GOMEZ-ROCA, C. A. et al.** Phase I study of emactuzumab single agent or in combination with paclitaxel in patients with advanced/metastatic solid tumors reveals depletion of immunosuppressive M2-like macrophages.. *Ann. Oncol.*, 2019, vol. 30, 1381-1392

- **RITTMEYER, A. et al.** Atezolizumab versus docetaxel in patients with previously treated non-small-cell lung cancer (OAK): a phase 3, open-label, multicentre randomised controlled trial.. *Lancet*, 2017, vol. 389, 255-265

- **CONNOLLY, J. G.** ; **SCHNEEWEISS, S.** ; **GLYNN, R. J.** ; **GAGNE, J. J.** Quantifying bias reduction with fixed-duration versus all-available covariate assessment periods.. *Pharmacoepidemiol. Drug Saf.*, 2019, vol. 28, 665-670
- **HOUWELINGEN, H. C. V.** Dynamic Prediction by Landmarking in Event History Analysis.. *Scand. J. Stat.*, 2007, vol. 34, 70-85